# EUROPEAN PATENT APPLICATION

(11) **EP 4 728 961 A1**
(43) Date of publication of application: **22.04.2026**
(21) Application number: 24863273.9
(22) Date of filing: 06.09.2024
(51) Int. Cl.: A61B 1/00, A61B 1/005

(54) **ENDOSCOPE**

(30) Priority: 08.09.2023 KR 20230119420; 08.09.2023 KR 20230119428
(71) Applicant: LG Electronics Inc., Yeongdeungpo-gu Seoul 07336 (KR)
(72) Inventor: HWANG, Changkyu, Seoul 06772 (KR); KIM, Wonsu, Seoul 06772 (KR); KIM, Soonbeom, Seoul 06772 (KR)
(74) Representative: Vossius & Partner Patentanwälte Rechtsanwälte mbB
(86) International application number: PCT/KR2024/013546
(87) International publication number: WO 2025/053694

(57) **Abstract**

Disclosed is an endoscope, including: an insertion part including a lighting imaging unit and an insertion tube; and a manipulation part including a manipulation knob and a manipulation wire configured to bend and operate the insertion tube according to manipulation of the manipulation knob, and the manipulation part may include: a coupling part coupled to the insertion part according to a rotation of a dial provided to be rotatably operable, thereby there is an effect that the operator may easily couple the insertion part and the manipulation part to each other through a simple manipulation of turning the dial.

## Description

### [BACKGROUND]

### [Technical Field]

Embodiments of the present invention relate to an endoscope, and more particularly, to an endoscope in which an insertion unit and an operating unit are detachably provided so that a disposable insertion unit can be replaced and used.

### [Background Art]

An endoscope for a medical use is an apparatus being directly inserted into an organ of a human body subject for an examination or treatment so as to check up a state of an affected area or to perform a treatment.

A general endoscope has an assembly body of a joint part disposed at an end of a flexible insertion tube and configured to adjust an insertion direction in the course of inserting the insertion tube. At an end of the assembly body of the joint part, a tip end body on which an image sensor and a light source are mounted is provided. At another end of the insertion tube, a handle body can be provided. The handle body is connected to an imaging device, which is not illustrated, through a separate cable or connector.

In the handle body, a dial and buttons used for adjusting a direction of the assembly body of the joint part and conducting a treatment are provided.

The dial is provided as a pair, and allows a heading direction of the tip end body to be adjusted in upward and downward directions and left and right directions with respect to a longitudinal direction of the insertion tube. Inside the handle body, a sprocket and a chain are provided so as to convert a rotation of the dial into a linear movement. The chain is connected to an end of the assembly body of the joint part through an additional wire. As a result, a rotation of the dial is converted into a linear movement of the wire through the sprocket and the chain, and the assembly body of the joint part is bent according to the linear movement of the wire. Accordingly, an operator of the endoscope becomes possible to adjust the heading direction of the tip end body while inserting the insertion tube into the human body.

The endoscope is inserted into the human body, and exhaustive hygiene control is needed, and must be washed and sterilized before and after a treatment or surgery. Depending on cases, it may be impossible or may not be desirable to reuse portions actually inserted into the human body, that are, the insertion tube and the assembly body of the joint part. In such a case, because the conventional endoscope is integrally formed as a whole body, there is a problem that the endoscope as a whole should be replaced. In addition, even if the reuse is possible, it would be easier to wash and sterilize the endoscope when it is possible to separate a portion only from the endoscope, rather than washing and sterilizing the entire endoscope.

Korea Patent No. 10-2014-0063947 discloses an endoscope configured to detach/attach a body of the manipulation part and the handle body. However, in the endoscope, separating and recoupling the wire is not easy due to the operational structure, and because leaving the wire as it is and making the other part be separated, the efficiency is low. Even if the problem of detaching/attaching the wire is solved, because distances that the pair of wires spaced apart from each other move were different due to a difference in a radius in a state in which the endoscope is bent, it is difficult to precisely manipulate the endoscope.

### [DETAILED DESCRIPTION OF THE INVENTION]

### [Technical Problem]

The present is thought of in order to improve above-described problems that the conventional endoscope had, and the purpose of the present invention is to provide an endoscope capable of allowing the user to easily couple the insertion part and the manipulation part to each other.

In addition, the purpose of the present invention is to provide an endoscope capable of allowing the wire of the insertion part and the wire of the manipulation part to be precisely coupled to each other.

### [Technical Solution]

One embodiment is an endoscope, including: an insertion part including a lighting imaging unit and an insertion tube; and a manipulation part including a manipulation knob and a manipulation wire configured to bend and operate the insertion tube according to manipulation of the manipulation knob, and the manipulation part may include: a coupling part coupled to the insertion part according to a rotation of a dial provided to be rotatably operable.

In more detail, the coupling part may be a first coupling part provided in the manipulation part.

At this instance, the coupling part may include a first coupling plate moved by a rotation of the dial to be coupled to the insertion part.

At this instance, the coupling part may further include: a pinion gear configured to be rotated in interrelation with the rotation of the dial; and a rack gear coupled to the first coupling plate and configured to be engaged with the pinion gear and to reciprocate according to a rotation of the pinion gear.

Meanwhile, the coupling part may further include: a first wire frame to which the manipulation wire is coupled and configured to reciprocate along the first coupling plate.

Therefore, when manipulating the manipulation knob, the first wire frame may be rectilinearly reciprocated along a guide groove formed in the first coupling plate.

Meanwhile, the coupling part may further include: a locking pin disposed on the first coupling plate and accommodated in a fixing groove formed on the first wire frame according to a reciprocation.

In addition, the coupling part may further include: a spring configured to pressurize the locking pin in a direction of the first wire frame.

In addition, the coupling part may further include: a locking holder coupled to the first coupling plate and accommodating the locking pin to be capable of reciprocating.

Meanwhile, the insertion part may include: a second coupling part coupled to the first coupling part.

At this instance, the second coupling part may include a second coupling plate detachably coupled to the first coupling plate.

The second coupling part may include: a second wire frame to which a bending wire is coupled, coupled to the first wire frame and configured to reciprocate together with the first wire frame.

Therefore, the first wire frame and the second wire frame may be coupled to each other, may rectilinearly move along a guide space formed by the first coupling plate and the second coupling plate coupled to each other, and may transfer a manipulation force of the manipulation knob.

That is, the manipulation part may include: a dial provided to be rotatably operable; a pinion gear configured to be rotated in interrelation with the rotation of the dial; a rack gear configured to be engaged with the pinion gear and to reciprocate according to a rotation of the pinion gear; and a coupling plate to which the rack gear is coupled and detachably coupled to the insertion part, therefore the manipulation part and the manipulation part may be detachably coupled to each other through a rotation of the dial.

The insertion part may include: a bending wire configured to bend and operate the insertion tube, and the manipulation part may include: a manipulation wire configured to move the bending wire according to manipulation of the manipulation knob, and the bending wire and the manipulation wire may be detachably connected to each other according to an operation of the dial.

### [Advantageous Effects]

As above-described, according to the endoscope of the present invention, there is an effect that the endoscope includes a pinion gear and a rack gear, and the user can easily couple the insertion part and the manipulation part to each other through a simple manipulation of turning a dial connected to the pinion gear.

In addition, there is an effect of precisely manipulating the wires because the endoscope includes a wire frame which connects the wire of the insertion part and the wire of the manipulation part to each other and allows the wires to rectilinearly reciprocate within the coupling plate.

### [Description of Drawings]

FIG. 1 is a perspective view for describing an endoscope according to an embodiment of the present invention.
FIG. 2 is a perspective view for describing an attachment and detachment relationship between a manipulation part and an insertion part according to an embodiment of the present invention.
FIG. 3 is a top view of FIG. 2.
FIG. 4 is a view for describing a coupling manner of a first coupling part and a second coupling part according to an embodiment of the present invention.
FIG. 5 is an enlarged view of FIG. 4 when viewed from a different angle.
FIG. 6 is a view for describing a coupled state in which a first coupling part and a second coupling part are coupled to each other in an endoscope according to an embodiment of the present invention.
FIGS. 7 to 13 are views for describing a structure guiding an initial position of a wire frame in an endoscope according to an embodiment of the present invention.
FIG. 14 is a perspective view of FIG. 2 when viewed from a different angle.
FIG. 15 is a perspective view for describing a lighting imaging unit in an endoscope according to an embodiment of the present invention.
FIG. 16 is a perspective view for describing an internal structure of a lighting imaging unit in an endoscope according to an embodiment of the present invention.
FIG. 17 is a view for describing a heat radiating plate in an endoscope according to an embodiment of the present invention.
FIGS. 18 and 19 are views for describing an insertion tube in an endoscope according to an embodiment of the present invention.
FIG. 20 is a view for describing a first bending member in an endoscope according to an embodiment of the present invention.
FIG. 21 is a view for describing a second bending member in an endoscope according to an embodiment of the present invention.

### [Description of Embodiments]

Hereinafter, exemplary embodiments of the present invention will be described in detail with reference to the accompanying drawings.

The present invention may be variously modified and may have various embodiments, and particular embodiments illustrated in the drawings will be specifically described below. The description of the embodiments is not intended to limit the present invention to the particular embodiments, but it should be interpreted that the present invention is to cover all modifications, equivalents and alternatives falling within the spirit and technical scope of the present invention.

In the description of the present invention, the terms such as "first" and "second" may be used to describe various components, but the components should not be limited by the terms. These terms are used only to distinguish one component from another component. For example, a first component may be named a second component, and similarly, the second component may also be named the first component, without departing from the scope of the present invention.

The term "and/or" may include any and all combinations of a plurality of the related and listed items.

When one component is described as being "coupled" or "connected" to another component, it should be understood that one component can be coupled or connected directly to another component, and an intervening component can also be present between the components. When one component is described as being "coupled directly to" or "connected directly to" another component, it should be understood that no intervening component is present between the components.

The terms used herein is used for the purpose of describing particular embodiments only and is not intended to limit the present invention. Singular expressions may include plural expressions unless clearly described as different meanings in the context.

The terms "comprises," "comprising," "includes," "including," "containing," "has," "having" or other variations thereof are inclusive and therefore specify the presence of stated features, integers, steps, operations, elements, and/or components, but do not preclude the presence or addition of one or more other features, integers, steps, operations, elements, components, and/or groups thereof.

Unless otherwise defined, all terms used herein, including technical or scientific terms, may have the same meaning as commonly understood by those skilled in the art to which the present invention pertains. The terms such as those defined in a commonly used dictionary may be interpreted as having meanings consistent with meanings in the context of related technologies and may not be interpreted as ideal or excessively formal meanings unless explicitly defined in the present application.

Further, the following embodiments are provided to more completely explain the present invention to those skilled in the art, and shapes and sizes of elements illustrated in the drawings may be exaggerated for a more apparent description.

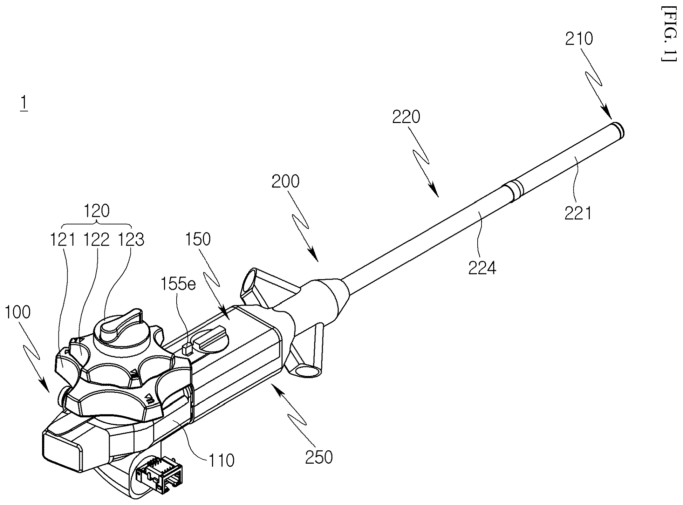

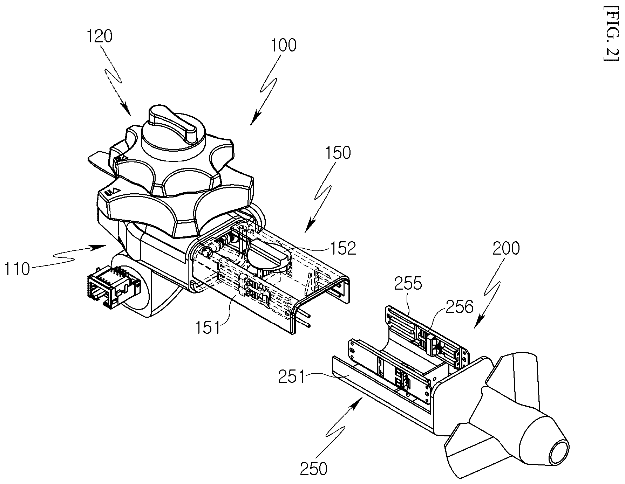

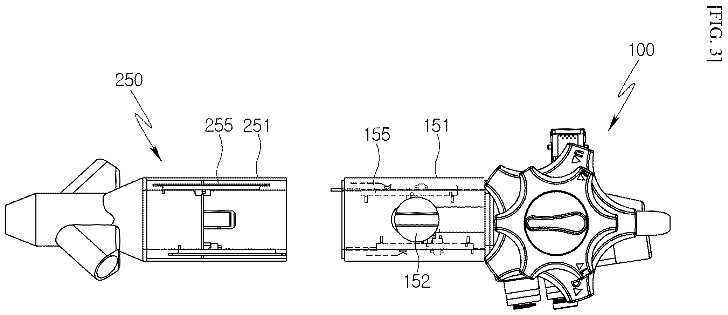

FIG. 1 is a perspective view for describing an endoscope according to an embodiment of the present invention, FIG. 2 is a perspective view for describing an attachment and detachment relationship between a manipulation part and an insertion part according to an embodiment of the present invention, and FIG. 3 is a top view of FIG. 2.

Referring to FIGS. 1 to 3, an endoscope 1 according to the present invention may be a disposable endoscope. In more detail, the endoscope 1 according to an embodiment of the present invention includes a manipulation part 100 and an insertion part 200. The manipulation part 100 and the insertion part 200 may be detachably coupled to each other by coupling parts 150 and 250. The coupling parts 150 and 250 include a first coupling part 150 provided in the manipulation part 100 and a second coupling part 250 provided in the insertion part 200.

The coupling parts 150 and 250 in the present invention are configured such that a rotation of a dial 152 is converted into a rectilinear movement by a pinion gear 153 and a rack gear 154 and accordingly, coupling plates 155 and 255 provided in the manipulation part 100 and the insertion part 200 are coupled to each other. The coupling parts 150 and 250 include a first coupling plate 155 provided in the manipulation part 100 and a second coupling plate 255 provided in the insertion part 200.

Meanwhile, in the present invention, connection of wires 130 and 230 can be performed by wire frames 156 and 256. That is, the wire 130 includes a manipulation wire 130 provided in the manipulation part 100 and a bending wire 230 provided in the insertion part 200, and the manipulation wire 130 and the bending wire 230 are coupled, respectively, to a first wire frame 156 provided in the manipulation part 100 and a second wire frame 256 provided in the insertion part 200. The first wire frame 156 and the second wire frame 256 are detachably coupled to each other and may rectilinearly reciprocate within the coupling plates 155 and 255 in the coupled state.

The above description will be described in greater detail below.

The manipulation part 100 is provided to be operable by the operator. The manipulation part 100 may be provided to be gripped by the operator, and to be operable by the operator. The manipulation part 100 may bend and operate the insertion part 200 through a manipulation of the operator.

The insertion part 200 is provided such that the manipulation part 100 is inserted into the human body by a manipulation of the manipulation part 100 so as to capture images of an inside of the human body. The insertion part 200 is provided to be inserted into the human body and to be bent by a manipulation of the manipulation part 100 so as to be moved along an internal structure of the human body, and is capable of emitting light to and capturing images of an inside of the human body.

Although not illustrated, except the above-described, the endoscope 1 according to the embodiment of the present invention may further include a connector adaptor and a universal cord.

In more detail, the endoscope 1 includes the connector adaptor to be connected to an external device, and the connector adaptor may be connected to the manipulation part 100 by the universal cord. The external device connected to the connector adaptor may be an image processing device. By doing so, an image captured by the insertion part 200 may be displayed in an external image processing device. With this configuration, the operator may manipulate the manipulation part 100 while watching the image captured by the insertion part 200 and displayed in the image processing device.

The manipulation part 100 includes a manipulation part housing 110, a manipulation knob 120, the manipulation wire 130, and the first coupling part 150.

The manipulation part housing 110 provides a structure which can be gripped by the operator and accommodates the manipulation wire 130 and the like configured to operate the insertion part 200.

In more detail, the manipulation knob 120 is disposed on an upper side of the manipulation part housing 110, the first coupling part 150 is disposed on one side in a longitudinal direction of the manipulation knob 120, and the sprocket, the chain, and the manipulation wire 130 are accommodated in the manipulation part housing 110, and a channel and the like may be further accommodated therein.

The manipulation knob 120 may control the insertion part 200 while being turned by a manipulation of the operator. The manipulation knob 120 may include two or more knobs. For example, the manipulation knob 120 may be provided in a stacked form in which a first knob 121 and a second knob 122 are stacked along a vertical direction. The first knob 121 and the second knob 122 are connected to a first sprocket and a second sprocket, respectively, and are configured such that each of which can rotate independently from each other. The first knob 121 and the second knob 122 have a form in which a plurality of teeth protrudes so that the operator can easily grip and rotate the knobs.

Meanwhile, the first knob 121 may independently rotate while maintaining a state in which the second knob 122 is stopped. Similarly, the second knob 122 may independently rotate while maintaining a state in which the first knob 121 is stopped. The first knob 121 controls a vertical movement of a lighting imaging unit 210. That is, when turning the first knob 121 in a clockwise or a counterclockwise direction, an end of the lighting imaging unit 210 is bent upward or downward accordingly. The second knob 122 controls a left or right movement of the lighting imaging unit 210. That is, when turning the second knob 122 in a clockwise or a counterclockwise direction, the end of the lighting imaging unit 210 is bent to the right or to the left accordingly.

As such, through the manipulation of the first knob 121 and the second knob 122, a rotational direction of the lighting imaging unit 210 can be controlled at discretion. According to the embodiment, it may be required to fix a state in which the lighting imaging unit 210 is bent at a certain angle, and to this end, an upper knob 123 is provided on an upper side of the second knob 122. When turning a fixing knob 123, the first and the second sprockets are fixed to be prevented from rotation, and accordingly, an end of the lighting imaging unit 210 is maintained at a fixed state.

Although not illustrated, two sprockets are rotatably provided below the first and the second knobs 121 and 122 so as to rotate in interrelation with a rotation of the first and the second knobs 121 and 122. In addition, a chain configured to move while engaged with each of the sprockets is provide. The chains are in charge of moving the lighting imaging unit 210 in upward and downward directions and left and right directions, and each of the chains can move independently from each other.

Each of both ends of the chain is coupled to manipulation wire 130. A movement of the manipulation wire 130 is guided through a guide structure formed inside the manipulation part housing 110.

The manipulation wire 130 is coupled to the first coupling part 150. In more detail, the manipulation wire 130 is coupled by the first wire frame 156 of the first coupling part 150. The first wire frame 156 is mounted to be capable of sliding in a limited range of the first coupling plate 155.

Meanwhile, the first wire frame 156 is coupled to the second wire frame 256 of the insertion part 200, and accordingly, the manipulation wire 130 can be connected to the bending wire 230.

Therefore, the manipulation wire 130 can bend and operate the insertion tube 220 according to the manipulation of the manipulation knob 120.

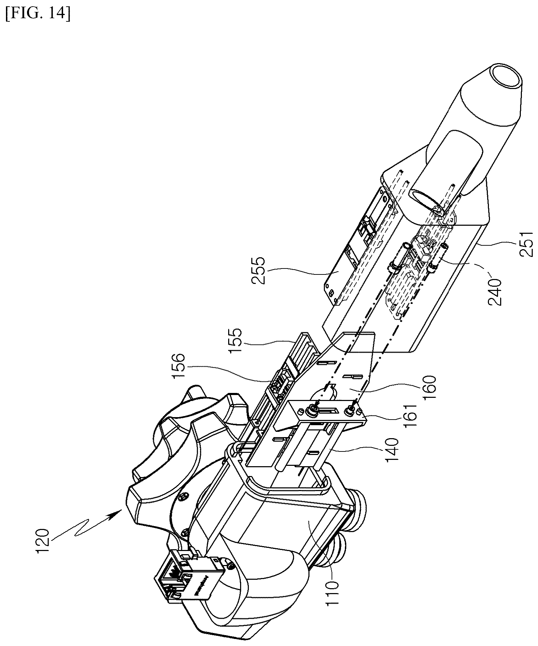

Meanwhile, a perspective view of FIG. 2 when viewed from a different angle is illustrated in FIG. 14.

Referring to FIG. 14, in the manipulation part 100, the channel 140 for providing air and water to be provided to the affected area when conducting a treatment may be provided. For example, the channel 140 may be a supply channel and a suction channel. At this time, the supply channel may be an air supply channel and/or a water supply channel. The air supply channel, the water supply channel, and the suction channel have approximately a cylindrical shape, and the inside thereof is empty so that when the channel is inserted into an inside of the lighting imaging unit 210 when coupled to the lighting imaging unit 210, water or air and the like is not leaked and can be stably supplied to the affected area or an area to be examined.

Meanwhile, although not illustrated, a connecting terminal may be further provided in the manipulation part 100. The connecting terminal may be electrically connected to the lighting imaging unit 210 so that data obtained by a camera and the like provided at an end of the lighting imaging unit 210 can be transmitted to an external device. For example, the connecting terminal may be a HDMI terminal for transmitting voice and images.

The channel 140 and the connecting terminal may be provided on a lower side than the wires 130 and 230. For example, the channel 140 and the connecting terminal may be accommodated in a space formed by the manipulation part housing 110, a first coupling part body 151, and a second coupling part body 251, and may be disposed to be farther from the manipulating knob 120 than the manipulation wire 130.

At this time, an area in which the manipulation wire 130 and the bending wire 230 move and an area in which the channel 140 and the connecting terminal are disposed may be distinguished by a support plate 160. The support plate 160 may partition an internal space of the manipulation part 100 in a vertical direction, has a channel support part 161 coupled to the channel 140 and supporting the channel 140, and therefore, may guide connection of the channel 140 at a correct position when the manipulation part 100 and the insertion part 200 are coupled to each other. That is, when the first coupling part 150 and the second coupling part 250 are coupled, the channel 140 of the manipulation part 100 may be connected to a channel 240 of the insertion part 200.

The channel support part 161 may extend downward from a lower surface of the support plate 160, and a circular hole may be formed in the channel support part 161 so that the channel 140 can be supported while passings therethrough. Here, a quantity of the hole may correspond to a quantity of the channel 140.

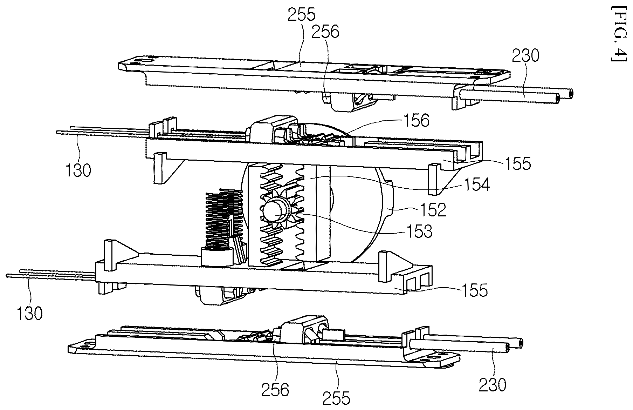

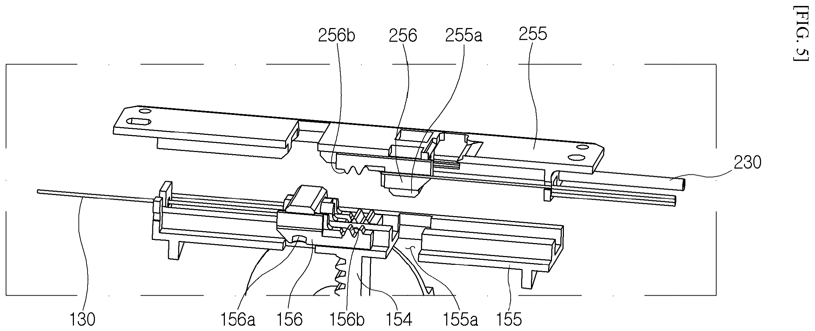

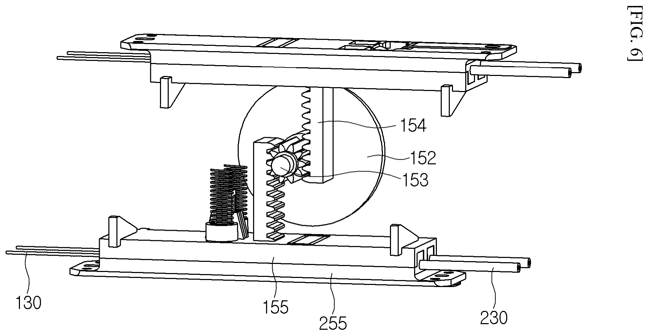

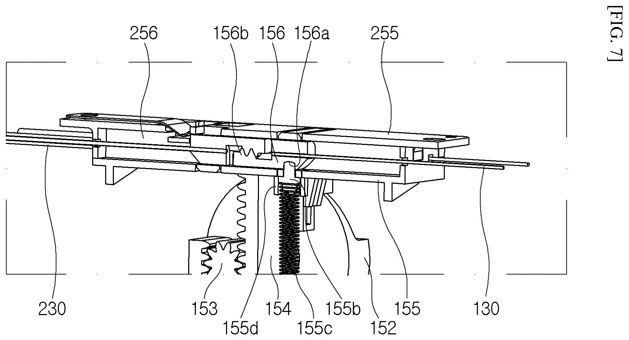

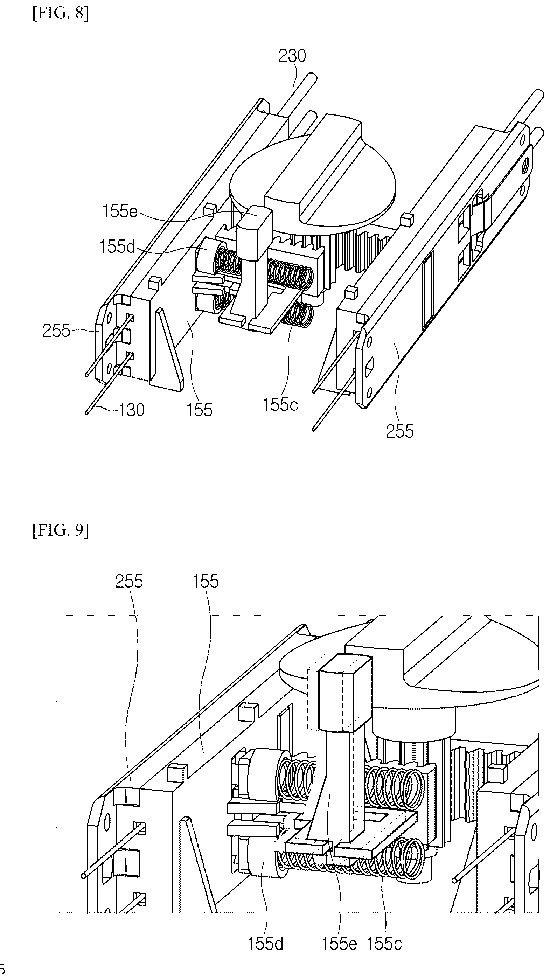

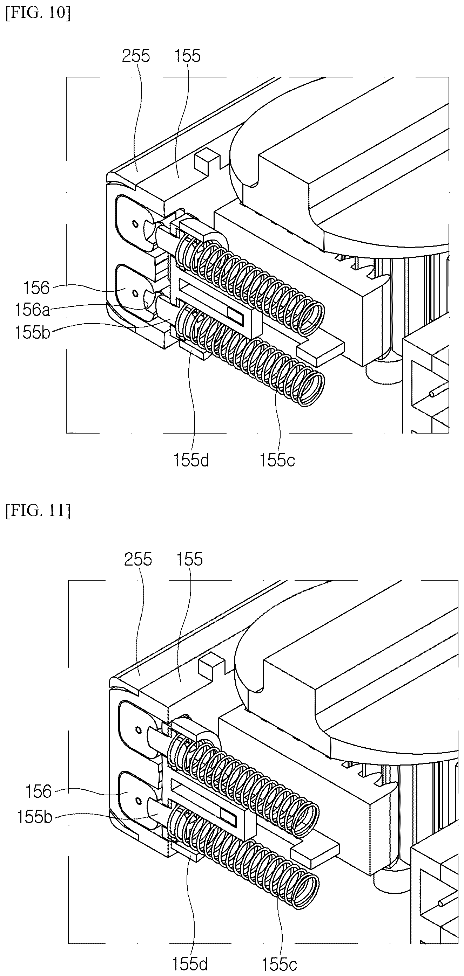

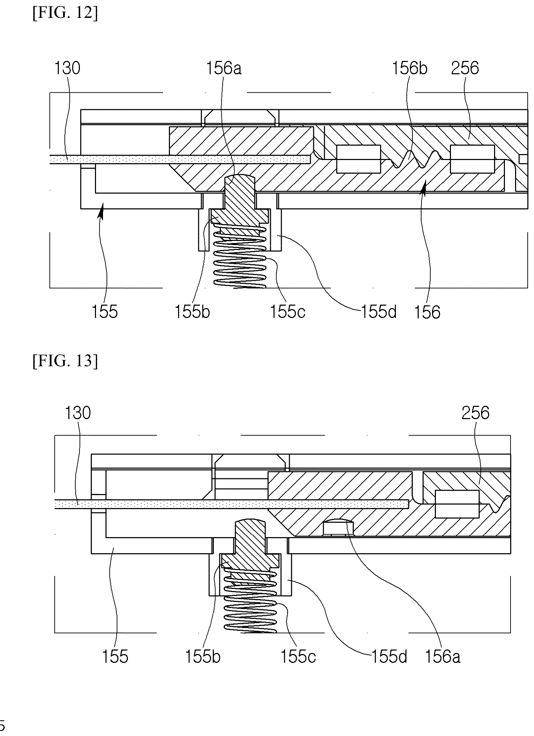

Meanwhile, FIG. 4 is a view for describing a coupling manner of the first coupling part and the second coupling part according to an embodiment of the present invention, FIG. 5 is an enlarged view of FIG. 4 when viewed from a different angle, FIG. 6 is a view for describing a coupled state in which the first coupling part and the second coupling part are coupled to each other in the endoscope according to an embodiment of the present invention, FIGS. 7 to 13 are views for describing a structure guiding an initial position of the wire frame in the endoscope according to an embodiment of the present invention.

Referring to FIGS. 4 to 13, the first coupling part 150 is described as below.

The first coupling part 150 may be disposed on one end of the manipulation part housing 110. The first coupling part 150 may be detachably coupled to the insertion part 200 by a manipulation of the operator (the user).

In more detail, the first coupling part 150 may include the first coupling part body 151, the dial 152, the pinion gear 153, the rack gear 154, and the first coupling plate 155.

The first coupling part body 151 is provided to be engaged with the second coupling part 250 of the insertion part 200. For example, the first coupling part body 151 may have a plate shape which surrounds three surfaces. As another example, the first coupling part body 151 may take a form of a pair of plates extending in a direction of the insertion part 200 from the manipulation part housing 110. Therefore, the first coupling part body 151 may be coupled to the second coupling part body 251 of the second coupling part 250.

As a result, the second coupling part body 151 and the second coupling part body 251 may be inserted into each other.

The dial 152 may be provided to be gripped and to be turned by the user. The dial 152 may be rotatably coupled to the manipulation part housing 110.

In more detail, the dial 152 may be exposed on the outside of the manipulation part housing 110. For example, the dial 152 may have a rotating plate having a round plate shape and a grip portion protruding in a rib shape from the rotating plate and allowing the user to grip and turn the dial 152.

The pinion gear 153 is disposed on an inside of the first coupling part body 151, and is rotated in interrelation with a rotation of the dial 152.

Meanwhile, the pinion gear 153 may be connected to the dial 152. The pinion gear 153 may pass through the first coupling part body 151 to be connected to the dial 152. For example, the pinion gear 153 and the dial 152 may be integrally formed.

Gear teeth are formed on an outer circumferential surface of the pinion gear 153, and the gear teeth may be engaged with the rack gear 154 which will be described below. Here, the pinion gear 153 may be engaged with a pair of rack gears 154. The pair of rack gears 154 may be disposed at positions facing each other based on the pinion gear 153.

The rack gears 154 are engaged with the pinion gear 153, and may be rectilinearly moved according to a rotation of the pinion gear 153. At this instance, the pair of rack gears 154 facing each other may be rectilinearly moved in directions opposite to each other according to a rotation of the pinion gear 153.

The pair of rack gears 154 may be disposed between a pair of the first coupling parts 151. Here, the pair of rack gears 154 may be disposed along a direction in which the first coupling part body 151 intersects a direction in which the first coupling part body 151 is formed, and may be rectilinearly reciprocated along a direction in which the first coupling part body 151 is formed.

Each of the pair of rack gears 154 may be coupled to the first coupling plate 155. The first coupling plate 155 may be formed along a direction intersecting a longitudinal direction of the rack gear 154.

The first coupling plate 155 may be disposed between a pair of surfaces, of the first coupling part body 151, facing each other. In addition, the first coupling plate 155 may be disposed between the second coupling part bodies 251 formed as a pair. Meanwhile, the first coupling plate 155 may be disposed on an inside of the second coupling part body 251, and may be disposed at a position facing the second coupling part body 251.

In addition, the first coupling plate 155 may be disposed at a position facing the second coupling plate 255. The first coupling plate 155 may be moved together with the rack gear 154 according to a movement of the rack gear 154 and be coupled to the second coupling plate 255.

For example, the first coupling plate 155 may have a quadrangular plate shape having a certain thickness. Here, in the first coupling plate 155, a fixing portion 155a may be formed so as to fix coupling between the first coupling plate 155 and the second coupling plate 255. For example, the fixing portion 155a may have a hole shape, and may be coupled to a fixing portion 255a having a protrusion shape and formed in the second coupling plate 255.

Meanwhile, in the first coupling plate 155, the first wire frame 156 may be disposed. The first wire frame 156 may be coupled to the first coupling plate 155 such that the first wire frame 156 can rectilinearly move. For example, on the first coupling plate 155, a guide groove for guiding the rectilinear movement of the first wire frame 156 may be formed, and the first wire frame 156 may be disposed in the guide groove.

Meanwhile, the manipulation wire 130 may be coupled to the first wire frame 156. For example, the manipulation wire 130 may be coupled to the first wire frame 156 on one side in a longitudinal direction, and the first wire frame 156 may be provided to be capable of rectilinearly moving along the longitudinal direction.

Meanwhile, the first wire frame 156, disposed on the first coupling plate 155, may be coupled to the second wire frame 256, disposed on the second coupling plate 255. For example, a frame coupling part 156b may be formed on another side in the longitudinal direction of the first wire frame 156. The frame coupling part 156b may have a shape in which a plurality of ribs and grooves are formed alternately. The frame coupling part 156b may have a shape corresponding to a shape of the frame coupling part 256b of the second wire frame 256. That is, the first wire frame 156 and the second wire frame 256 may be coupled to each other in a state of being engaged with each other.

The first wire frame 156 in a state of being coupled to the second wire frame 256 may be disposed between the first coupling plate 155 and the second coupling plate 255. Therefore, the first wire frame 156 and the second wire frame 256 in a state in which both are coupled to each other may rectilinearly move along a longitudinal direction of the endoscope 1.

With this configuration, the manipulation wire 130 coupled to the first wire frame 156, and the bending wire 230 coupled to the second wire frame 256 may be moved in interrelation with each other, and may bend the insertion tube 220 according to a manipulation of the manipulation knob 120.

Meanwhile, the first coupling plate 155 may further include a locking pin 155b, a spring 155c, and a locking holder 155d.

The locking pin 155b is disposed in the first coupling plate 155, and may be accommodated in a fixing groove 156a formed in the first wire frame 156, according to the reciprocation. The locking pin 155b may include a head portion accommodated in the locking holder 155d which will be described below, and a pin portion passing through the locking holder 155d and accommodated in the fixing groove 156a. Here, the head portion may have a shape having a greater diameter than that of the pin portion. Therefore, a movement range of the locking pin 155b may be limited by the locking holder 155d.

Meanwhile, the locking pin 155b may be pressurized toward a direction of the first wire frame 156 by the spring 155c. In more detail, the head portion of the locking pin 155b may be in contact with one end of the spring 155c. Therefore, the locking pin 155b may be elastically supported by the spring 155c.

The other end of the spring 155c may be fixed to the first coupling part body 151.

The locking holder 155d may be detachably coupled to the first coupling plate 155, and may accommodate the locking pin 155b such that the locking pin 155b can reciprocate.

The locking holder 155d may be coupled to the locking lever 155e, and may be reciprocated by an operation of the locking lever 155e. At this time, the locking holder 155d may be detachably coupled to the first coupling plate 155 according to an operation of the locking lever 155e.

The locking holder 155d may be formed such that an inside of the locking holder 155d can accommodate the locking pin 155b. For example, the locking holder 155d may be formed to have a cylindrical shape, and one side thereof in a longitudinal direction may be formed to correspond to a size of the head portion of the locking pin 155b, and the other side thereof in a longitudinal direction may be formed to correspond to a size of the pin portion of the locking pin 155b. That is, one side of the locking holder 155d in a longitudinal direction may be formed to be greater than a diameter of the head portion, and the other side of the locking holder 155d in a longitudinal direction may be formed to be smaller than the diameter of the head portion, but to be greater than a diameter of the pin portion.

Therefore, the pin portion of the locking pin 155b may pass through the locking holder 155d to be accommodated in the fixing groove 156a of the first wire frame 156.

For example, the locking holder 155d may be provided as a pair, and between the pair of locking holders 155d, a lever coupling portion to be coupled to the locking lever 155e may be provided. The lever coupling portion 155e may have a long hole shape, and one side of the locking lever 1553 may be coupled to the lever coupling portion 155e.

One side of the lever coupling portion 155e may be coupled to the locking holder 155d, and the other side thereof may be exposed to the outside of the first coupling part body 151. Therefore, the user may hold and manipulate the locking lever 155e.

Meanwhile, the fixing groove 156a may be formed in the first wire frame 156. The fixing groove 156a may be formed on an opposite surface to a surface on which the frame coupling part 156b is formed. That is, the frame coupling part 156b may be formed on a surface facing the second wire frame 256, and the fixing groove 156a may be formed on the opposite surface thereof.

The fixing groove 156a may accommodate the locking pin 155b according to a movement of the first wire frame 156. That is, when the first wire frame 1156 is disposed at a predetermined initial position, the fixing groove 156a may be disposed to face the locking pin 155b. At this time, when the user manipulates the locking lever 155e, the locking pin 155b is moved along the locking holder 155d, passes through the first coupling plate 155, and is inserted into the fixing groove 156a.

Therefore, according to the present invention, after replacing the insertion part 200, the user may turn the manipulation knob 120 in a state in which the locking lever 155e is manipulated by the user, and therefore, the first wire frame 156 is rectilinearly moved (slid), thereby finding a position at which the locking pin 155b and the fixing groove 156a are coupled.

As a result, according to the present invention, there is an effect of being capable of finding an initial position of the manipulation wire 130 after replacing the insertion part 200, and maintaining the manipulation wire 130 at the initial position.

The insertion part 200 may be detachably coupled to the manipulation part 100, and at least some of the insertion part 200 may be inserted into and capture images of an inside of the human body.

The insertion part 200 may include the lighting imaging unit 210, the insertion tube 220, the bending wire 230, and the second coupling part 250. At this time, on one side of the insertion tube 200 in the longitudinal direction, the lighting imaging unit 210 is disposed, and on the other side of the insertion tube 200 in the longitudinal direction, the second coupling part 250 is disposed. In addition, the lighting imaging unit 210 and the second coupling part 250 may be connected to each other through the insertion tube 220, and at least some of the bending wire 230 may pass through a portion from the second coupling part 250 to the insertion tube 220.

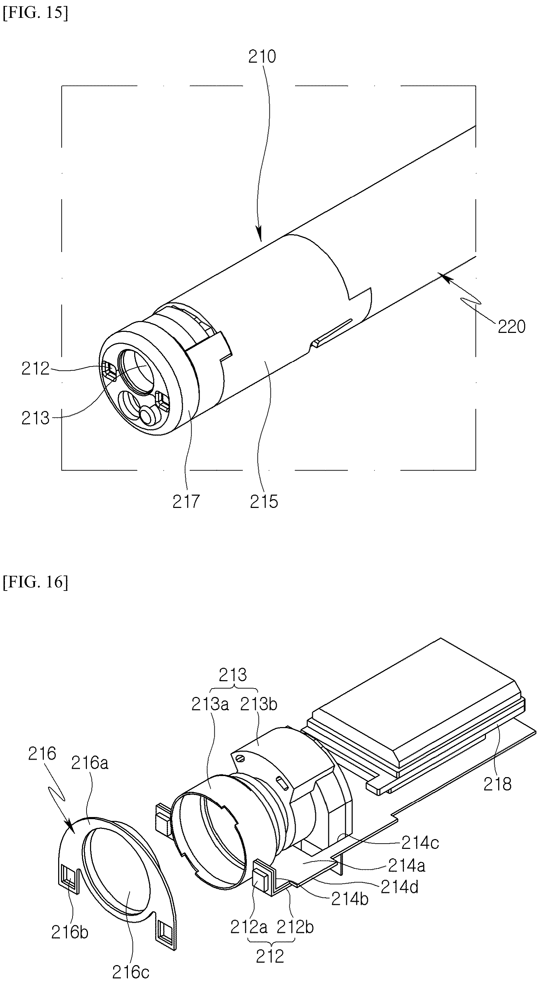

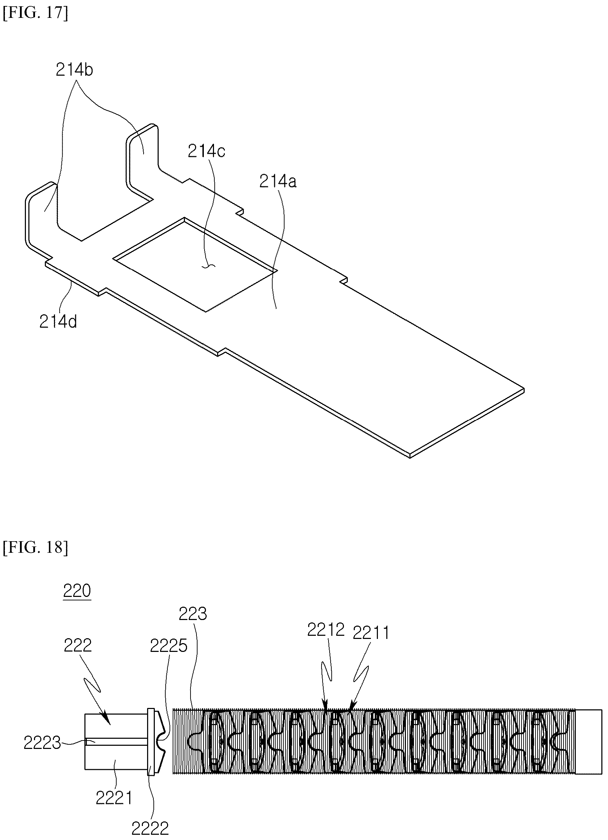

Meanwhile, FIG. 15 is a perspective view for describing the lighting imaging unit in the endoscope according to an embodiment of the present invention, FIG. 16 is a perspective view for describing an internal structure of the lighting imaging unit in the endoscope according to an embodiment of the present invention, and FIG. 17 is a view for describing a heat radiating plate in the endoscope according to an embodiment of the present invention.

Referring to FIGS. 15 to 17, the lighting imaging unit will be described as below.

The lighting imaging unit 210 is inserted into an inside of the human body to emit light and capture images of the inside.

The lighting imaging unit 210 includes a lighting imaging unit body 211, a lighting module 212, and an imaging module 213.

The lighting imaging unit body 211 defines an external appearance of the lighting imaging unit 210, and is configured to be inserted into the inside of the human body. For example, on one side of the lighting imaging unit body 211 in the longitudinal direction, the lighting module 212, and the imaging module 213 are disposed, and the other side of the lighting imaging unit body 211 in the longitudinal direction is opened and can be connected to the insertion tube 220.

The lighting module 212 may be coupled to an end cap 217. The lighting module 212 may emit light. The lighting module 212 may include a lighting lens 212a which receives power and emits light. For example, the lighting lens 212a may be an LED (Light Emitting Diode).

Meanwhile, in the present embodiment, the lighting lens 212a may be provided in plural number. In more detail, in the present embodiment, the lighting lens 212a may be disposed in an even number. For example, a pair of the lighting lenses 212a may be disposed to be linearly symmetrical. With this configuration, there is an effect of preventing the illuminance illuminated to an object in a short distance from being asymmetrical and preventing an optical vacancy of the lighting.

At this instance, the pair of lighting lenses 212a in the present embodiment may be disposed to be symmetrical based on the imaging modul3 213. That is, in the present embodiment, the pair of lighting lenses 212a are disposed to be symmetrical and the imaging module 213 may be disposed on a symmetrical axis.

Here, the pair of lighting lenses 212a may have different correlated color temperatures (CCT). With this configuration, targeted wavelength distribution of light can be made.

In addition, the lighting lens 212a may further include a lighting support portion 212b for supporting the pair of lighting lenses 212a. The lighting support portion 212b has a flat plate shape and tip ends as a pair thereof may extend in a symmetrical manner and be bent upward. To the pair of extending portions which are bent, the pair of lighting lenses 212a may be coupled.

For example, the lighting support portion 212b may be a flexible printed circuit board (PCB). On the flexible PCB, an image sensor may be mounted. There is an effect that the clarity of an image obtained through the image sensor can be further improved because the present embodiment uses a plurality of lighting lenses 212a as a lighting part, thus, control of the illuminance can be made easy and a light amount being emitted can be dramatically increased compared to a light amount of the existing halogen lamp and the like.

Meanwhile, the imaging module 213 may capture images inside the human body. The imaging module 213 may capture images inside the human body by receiving power and transmit the captured image data. For example, the imaging module 213 may include a camera 213a and an image processing portion 213b. Here, the camera 213a may be disposed on one end (tip end) in a longitudinal direction of the lighting imaging unit 210, and the image processing portion 213b may be coupled to the camera 213a, and may process images coming in from the camera.

With this configuration, in a state in which the endoscope 1 is inserted into the human body, the endoscope 1 of the present invention may emit light through the lighting module 212, and capture images of the inside of the human body through the imaging module 213.

A heat radiating plate 214 may be disposed inside the lighting imaging unit body 211. The heat radiating plate 214 may be coupled to the lighting module 212 and/or the imaging module 213.

The heat radiating plate 214 may include a heat radiating plate body 214a, a lighting contact portion 214b, an imaging module accommodation hole 214c, and a heat transferring portion 214d.

The heat radiating plate body 214a may be provided to transfer heat. The heat radiating plate body 214a may be formed of a material capable of transferring heat. For example, the heat radiating plate body 214a may be formed of a metal material.

The heat radiating plate body 214a may have a shape of a flat plate, with one end in the longitudinal direction (a long axis direction) connected to the lighting contact portion 214b, and both ends in a width direction (a short axis direction) thereof connected to the heat transferring portion 214d, and the imaging module accommodation hole 214c may be formed in the heat radiating plate body 214a.

The lighting contact portion 214b may extend from one end of the heat radiating plate body 214a in the longitudinal direction and may contact the lighting module 212. The lighting contact portion 214b may may be formed in correspondence with a shape of the lighting support portion 212b. For example, the lighting contact portion 214b may extend from a tip end in the longitudinal direction of the heat radiating plate body 214a and be bent and extend upward therefrom. Therefore, the lighting contact portion 214b may be in contact with the lighting support portion 212b. With this configuration, in the lighting contact portion 214b, heat generated in the lighting module 212 may be transferred to the heat radiating plate 214.

The imaging module accommodation hole 214c may be formed in the heat radiating plate body and accommodate the imaging module 213 therein to allow the imaging module 213 to pass therethrough. For example, the imaging module accommodation hole 214c may have a shape of a rectangular hole, with a portion of the image processing portion 213b passing therethrough, and a portion of the image processing portion 213b in contact therewith. With this configuration, heat generated in the imaging module 213 may be transferred to the heat radiating plate 214.

Meanwhile, a printed circuit board 218 may contact the other side in the longitudinal direction of the heat radiating plate 214. The printed circuit board 218 may control the lighting module 212 and/or the imaging module 213. On the printed circuit board 218, a circuit capable of controlling the lighting module 212 and/or the imaging module 213 may be mounted. With this configuration, heat generated in the circuit may be transferred to the heat radiating plate 214.

The heat transferring portion 214d may extend from both ends in a width direction (a short axis direction) of the heat radiating plate body 214a. At this time, a width of the heat radiating plate body 214a may be smaller than a diameter of a heat radiating chassis 215, and a width obtained by adding up the heat radiating plate body 214a and the heat transferring portion 214d may be equal to or lightly greater than the diameter of the heat radiating chassis 215. Therefore, the heat transferring portion 214d may be in contact with the heat radiating chassis 215. Accordingly, heat of the heat transferring portion 214d may be transferred to the heat radiating chassis 215.

The heat radiating chassis 215 may be provided to surround an outer circumferential surface of the lighting imaging unit body 211, and may be in contact with the heat radiating plate 214, thereby becoming able to distribute heat of the lighting module 212 and/or the imaging module 213.

For example, the heat radiating chassis 215 may be a coil pipe formed of a spring material, and may dissipate heat generated in the lighting module 212 and/or the imaging module 213.

Meanwhile, in another embodiment of the present invention, the heat radiating chassis 215 and the lighting imaging unit body 211 may be integrally formed.

Meanwhile, the lighting imaging unit 210 may further include a heat blocking plate 216 disposed on one side in the longitudinal direction of the lighting imaging unit 210, and configured to block heat generated in the lighting module 212.

The heat blocking plate 216 may be coupled to the end cap 217 and may be disposed between the end cap 217 and the lighting module 212 along the longitudinal direction of the lighting imaging unit 210.

The heat blocking plate 216 may include a blocking plate body 216a, lighting cover portions 216b disposed symmetrically in the blocking plate body 216a and disposed to face the lighting module 212, and a camera cover portion 216c disposed on a symmetrical axis of the pair of lighting cover portions 216b and disposed at a position facing the imaging module 213.

The heat blocking plate 216 may prevent heat generated in the lighting module 212 from being transferred to the human body. In this case, the heat blocking plate 216 may be formed of a light transmissive material.

On a tip end in the longitudinal direction of the lighting imaging unit 210, the end cap 217 is provided. The end cap 217 has a cylindrical shape, and is coupled to an end of the insertion part 200. At this instance, the lighting module 212 and the imaging module 213 may be coupled to the end cap 217. In addition, the heat blocking plate 216 may be coupled to the end cap 217.

Transparent windows may be provided in a plurality of portions in the end cap 217. The transparent windows are implemented as a sheet made of glass or a transparent material, and have a structure able to transfer light emitted from the lighting module 212.

Meanwhile, a plurality of channel receivers allowing the channel 240 to be inserted thereinto may be formed in the end cap 217. The respective channel receivers have the shape of a hollow cylinder, and are configured such that the inner diameters thereof correspond to the outer diameters of the channels 140 to be inserted thereinto. In addition, an O-ring preventing the leakage of air, water, or the like to be supplied may be provided inside each of the channel receivers.

A connecting terminal may be provided in the lighting imaging unit 210. The connecting terminal may be provided to correspond to the connecting terminal of the manipulation part 100. That is, while a male terminal is provided in the manipulation part 100, a female terminal may be provided in the insertion part 200.

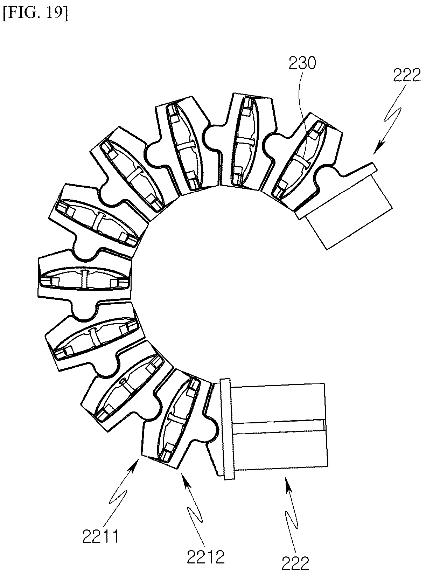

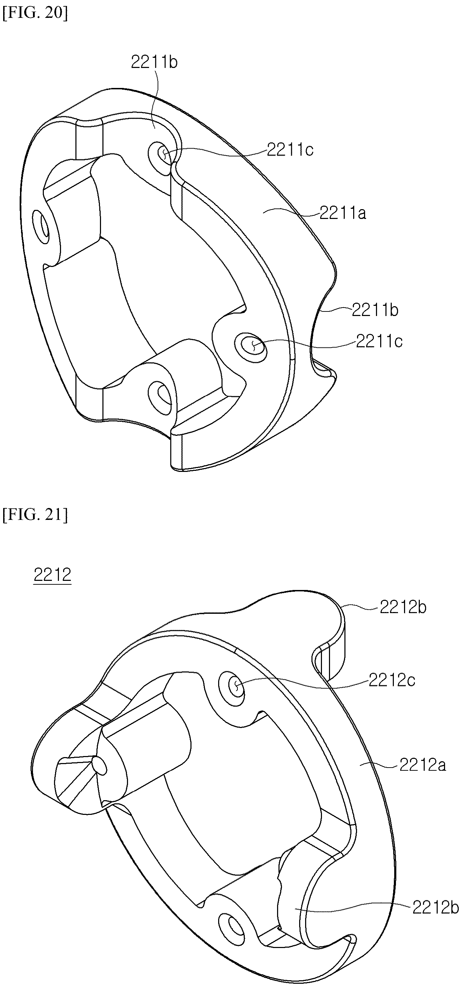

Meanwhile, FIGS. 18 and 19 are views for describing the insertion tube in the endoscope according to an embodiment of the present invention, FIG. 20 is a view for describing a first bending member in the endoscope according to an embodiment of the present invention and FIG. 21 is a view for describing a second bending member in the endoscope according to an embodiment of the present invention.

Referring to FIGS. 18 to 21, the insertion tube 220 will be described as below.

The insertion tube 220 may connect the lighting imaging unit 210 and the second coupling part 250 to each other, and may be bent according to the manipulation of the manipulation part 100.

In more detail, the insertion tube 220 may be bent according to a movement of the bending wire 230.

Meanwhile, a plurality of channels 240 and terminals may be accommodated in the insertion tube 220.

The insertion tube 220 may include a bending part 221, an adapter 222, a coil spring 223, and an insertion tube part 224. At this time, the bending part 221 may be disposed between a pair of adapters 222, and one among the pair of adapters 222 may be coupled to the lighting imaging unit 210, and the other thereof may be coupled to the second coupling part 250.

The bending part 221 may be configured to be bent according to the movement of the bending wire 230.

The bending part 221 may include a first bending member 2211 and a second bending member 2212, each of which rotating relative to each other. Here, the bending part 221 may be configured such that a plurality of first bending members 2211 and a plurality of second bending members 2212 are disposed alternately therein. In addition, as the quantity of the first bending members 2211 and the second bending members 2212 increases, a length of the entire bending part 221 may increase. Therefore, according to the use condition of the endoscope 1, it is possible to select a suitable length of the insertion tube 220 by adjusting the quantity of the first bending members 2211 and the second bending members 2212.

The first bending member 2211 and the second bending member 2212 may be provided to be able to rotate relative to each other.

The first bending member 2211 may include a first bending member body 2211a, a concave portion 2211b, and a wire through hole 2211c.

The first bending member body 2211a may entirely be formed in an annular shape. Here, a thickness in a longitudinal direction of the first bending member body 2211a may repeat changes at a certain degree interval along a circumferential direction. For example, the first bending member body 2211a may be formed to have a thickness which is being reduced as away from the concave portion 2211b along a circumferential direction.

Meanwhile, in the present embodiment, the first bending member body 2211a may be formed of a resin material. For example, the first bending member body 2211a may be formed of a plastic material. By doing so, there is an effect that formability is improved and production costs are reduced.

Here, in the first bending member body 2211a, the concave portion 2211b may be formed to be able to rotate relative to the second bending member 2212.

Four concave portions 2211b may be formed in the first bending member body 2211a, with a pair thereof formed on one end in the longitudinal direction of the first bending member body 2211a, and a pair thereof formed on the other end in the longitudinal direction of the first bending member body 2211a. The pair thereof formed on one end in the longitudinal direction of the first bending member body 2211a, and the pair thereof formed on the other end in the longitudinal direction of the first bending member body 2211a may be disposed alternately with a phase difference of 90 degrees from each other along a circumferential direction. That is, the pair of concave portions 2211b formed on one end in the same direction are formed with a phase difference of 180 degrees from each other along a circumferential direction, and the pair of concave portions 2211b formed on one end in the same direction and the pair of concave portions 2211b formed on the other end in the other direction may have a phase difference of 90 degrees with respect to each other.

The concave portion 2211b may be recessed from an end in the longitudinal direction of the first bending member body 2211a. Here, the concave portion 2211b may be recessed to take the form of an arc having a certain curvature from the end in the longitudinal direction of the first bending member body 2211a.

The concave portion 2211b may accommodate at least some of a convex portion 2212b of the second bending member 2212 which will be described below. In addition, the concave portion 2211b and the convex portion 2212b may be rotated relative to each other. At this instance, the concave portion 2211b and the convex portion 2212b may contact each other.

Therefore, in a state in which the first bending member body 2211a and a second bending member body 2212a are coupled, a spacing between the first bending member body 2211a and the second bending member body 2212a may not be regular. That is, when the first bending member body 2211a and the second bending member body 2212a are coupled, the concave portion 2211b and the convex portion 2212b may contact each other, and as away from the concave portion 2211b and the convex portion 2212b along the circumferential direction, the spacing between the first bending member body 2211a and the second bending member body 2212a may increase.

With this spacing, it is possible to provide a range in which the first bending member 2211 and the second bending member 2212 are rotated relative to each other according to the movement of the bending wire 230.

Meanwhile, in the first bending member body 2211a, a thickness in a radial direction of a position at which the concave portion 2211b is formed may increase. For example, the first bending member body 2211a may be formed to have a regular outer diameter on a concentric circle, however, an internal diameter of the first bending member body 2211a at a position at which the concave portion 2211b is formed may be formed to decrease. That is, the first bending member body 2211a may have a shape of which an inner circumferential surface at a position at which the concave portion 2211b is formed protrudes radially inward.

In addition, at the position at which the concave portion 2211b is formed, the wire through hole 2211c may be formed along the longitudinal direction. That is, the wire through hole 2211c may be a hole in a circular shape formed at a position protruding radially inward, on the inner circumferential surface of the first bending member body 2211a.

At this instance, the bending wire 230 may pass through the wire through hole 2211c.

The second bending member 2212 includes the second bending member body 2212a, the convex portion 2212b, and a wire through hole 2212c.

The second bending member body 2212a may entirely be formed in an annular shape. Here, a thickness in a longitudinal direction of the second bending member body 2212a may repeat changes at a 180 degrees interval along a circumferential direction. For example, the second bending member body 2212a may be formed to have a longitudinal thickness which is being reduced as away from a position at which the convex portion 2212b is formed along a circumferential direction.

Meanwhile, in the present embodiment, the second bending member body 2212a may be formed of a resin material. For example, the second bending member body 2212a may be formed of a plastic material. By doing so, there is an effect that formability is improved and production costs are reduced.

Here, in the second bending member body 2212a, the convex portion 2212b may be formed to be able to rotate relative to the first bending member 2211.

Four convex portions 2212b may be formed in the second bending member body 2212a, with a pair thereof formed on one end in the longitudinal direction of the second bending member body 2212a, and a pair thereof formed on the other end in the longitudinal direction of the second bending member body 2212a. The pair thereof formed on one end in the longitudinal direction of the second bending member body 2212a, and the pair thereof formed on the other end in the longitudinal direction of the second bending member body 2212a may be disposed alternately with a phase difference of 90 degrees from each other along a circumferential direction. That is, the pair of convex portions 2212b formed on one end in the same direction are formed with a phase difference of 180 degrees from each other along a circumferential direction, and the pair of convex portions 2212b formed on one end in the same direction and the pair of convex portions 2212b formed on the other end in the other direction may have a phase difference of 90 degrees with respect to each other.

The convex portion 2212b may protrude from an end in the longitudinal direction of the second bending member body 2212a. Here, the convex portion 2212b may be formed in correspondence with a shape of the concave portion 2211b. For example, the convex portion 2212b may protrude, in the form of an arc having a certain curvature, from the end in the longitudinal direction of the second bending member body 2212a, and a curvature of the protrusion of the convex portion 2212b and a curvature of the recess of the concave portion 2211b may be the same.

With this configuration, at least some of the convex portion 2212b may be accommodated in the concave portion 2211b. In addition, the convex portion 2212b and the concave portion 2211b may be rotated relative to each other while being in surface contact with each other.

Meanwhile, in the second bending member body 2212a, a thickness in a radial direction of a position at which the convex portion 2212b is formed may increase. For example, the second bending member body 2212a may be formed to have a regular outer diameter on a concentric circle, however, an internal diameter of the second bending member body 2212a at a position at which the convex portion 2212b is formed may be formed to decrease. That is, the second bending member body 2212a may have a shape of which an inner circumferential surface at a position at which the convex portion 2212b is formed protrudes radially inward.

In addition, at the position at which the convex portion 2212b is formed, the wire through hole 2212c may be formed along the longitudinal direction. That is, the wire through hole 2212c may be a hole in a circular shape formed at a position protruding radially inward, on the inner circumferential surface of the second bending member body 2212a.

At this instance, the bending wire 230 may pass through the wire through hole 2212c.

Meanwhile, an inner circumferential surface of the convex portion 2212b may not protrude radially inward, but may be formed to be the same as the maximum internal diameter of the second bending member body 2212a. That is, the inner circumferential surface of the convex portion 2212b may not protrude radially inward more than a position at which the wire through hole 2212c is formed. Therefore, when viewing the inner circumferential surface of the second bending member 2212 as whole, the inner circumferential surface is regular, but the inner circumferential surface at a position at which the wire through hole 2212c is formed has a shape protruding radially inward.

In addition, in the inner circumferential surface of the convex portion 2212b, a wire guide groove 2212d may be formed. The wire guide groove 2212d may be recessed in an arc shape on the inner circumferential surface of the convex portion 2212b, but the origin of the arc may be connected to the wire through hole 2212c.

With this configuration, when the bending wire 230 moves with a fixed end, which is a position contacting the second bending member body 2212a, a movement range of a free end may be limited by the wire guide groove 2212d. That is, the maximum angle at which the second bending member 2212 is bent may be a center angle of the arc of the wire guide groove 2212d.

The adapters 222 may be coupled to both ends in the longitudinal direction of the bending part 221. Here, the adapter 222 disposed on one side in the longitudinal direction may be coupled to the lighting imaging unit 210, and the adapter 222 disposed on the other side in the longitudinal direction may be coupled to the second coupling part 250.

The adapter 222 includes an adapter body 2221, a spring coupling portion 2222, a wire accommodation groove 2223, a wire through hole, and a bending member connecting portion 2225.

The adapter body 2221 may have a cylindrical shape as a whole, and the spring coupling portion 222 may be formed on an outer circumferential surface of the adapter body 2221. In addition, on an outer circumferential surface, or an inner circumferential surface of the adapter body 2221, the wire accommodation groove 2223 may be formed along the longitudinal direction. Further, the wire through hole may be formed along the longitudinal direction in the adapter body 2221. Moreover, the bending member connecting portion 2225 may be formed in an end in the longitudinal direction of the adapter body 2221.

An end in the longitudinal direction of the coil spring 223 may be coupled to the spring coupling portion 2222. For example, the spring coupling portion 2222 may have a shape of a rib protruding along the circumferential direction from the outer circumferential surface of the adapter body 2221. With this configuration, the end of the coil spring 223 may be caught and be supported by the spring coupling portion 2222.

Therefore, the coil spring 223 may be disposed between the spring coupling portions 2222 formed in the pair of adapters 222, and a restorative force of the coil spring 223 may be applied to the pair of adapters 222.

The wire accommodation groove 2223 may be formed along the longitudinal direction on an outer circumferential surface, or an inner circumferential surface of the adapter body 2221. For example, four wire accommodation grooves 2223 may be formed along the longitudinal direction on an inner circumferential surface of the adapter body 2221.

At least some of the bending wire 230 may be accommodated in the wire accommodation groove 2223. The bending wire 230 may reciprocate in the longitudinal direction along the wire accommodation groove 2223 according to the manipulation of the manipulation knob 120.

Although not illustrated, the wire through hole may be formed along the longitudinal direction of the adapter body 2221. Here, at least some of the inner circumferential surface of the adapter body 2221 protrudes at a position at which the wire accommodation groove 2223 is formed, and the wire through hole may be formed at the protruding position. That is, the wire through hole may be disposed at a position which is connected to the wire accommodation groove 2223.

At this instance, the bending wire 230 may pass through the wire through hole.

Meanwhile, the bending member connecting portion 2225 may be formed to be coupled to the bending part 221 in a manner capable of rotating relative to the bending part 221. That is, the bending member connecting portion 2225 may have a convex or concave structure corresponding to the concave portion 2211b of the first bending member 2211 or the convex portion 2212b of the second bending member 2212 so as to be able to be coupled thereto.

Meanwhile, the coil spring 223 may be disposed to surround an outer circumferential surface of the bending part 221. That is, in a state in which the first bending member 2211 and the second bending member 2212 are coupled, the coil spring 223 may be formed to surround the outside of the outer circumferential surfaces of the first bending member 2211 and the second bending member 2212.

On contrary, both ends in the longitudinal direction of the coil spring 223 may be coupled to the adapter 222. The coil spring 223 may be elastically supported by the spring coupling portions 2222 of the pair of the adapters 222. Therefore, the coil spring 223 may apply an elastic force to return the bending part 221 to its original position, when the bending part 221 is bent according to the manipulation of the manipulation knob 120.

Meanwhile, on the other side in the longitudinal direction of the insertion tube 220, the insertion tube part 224 may be disposed. The insertion tube part 224 may have a form of a bendable tube, and the bending wire 230, the channel 240, and the terminal may pass therethrough. Although not illustrated, a coil pipe is provided inside the insertion tube part 224 so as to provide an elastic force to the insertion tube part 224.

Meanwhile, the bending wire 230 is applied with a manipulation force of the manipulation knob 120 through the second coupling part 250, and may bend and operate the insertion tube 220 through the rectilinear reciprocation.

One end in the longitudinal direction of the bending wire 230 is coupled to the adapter 222 or the lighting imaging unit 210, and the other end of the longitudinal direction of the bending wire 230 is coupled to the second wire frame 256. At this instance, the bending wire 230 may pass through the wire through hole of the adapter 222, the wire through hole 2211c of the first bending member 2211, and the wire through hole 2212c of the second bending member 2212.

Therefore, on a side on which the bending wire 230 is pulled, the first bending member 2211 and the second bending member 2212 may rotate relative to each other such that a spacing between the first bending member 2211 and the second bending member 2212 is reduced. On contrary, on a side on which the bending wire 230 is released (elongated), the first bending member 2211 and the second bending member 2212 may rotate relative to each other such that a spacing between the first bending member 2211 and the second bending member 2212 is increased. Accordingly, the first bending member 2211 and the second bending member 2212 may form a bent shape. At this instance, an angle of the bending between the first bending member 2211 and the second bending member 2212 may be limited according to an angle of the wire guide groove 2212d.

Meanwhile, referring to FIGS. 4 to 13, the second coupling part 250 is detachably coupled to the first coupling part 150. The second coupling part 250 includes the second coupling part body 251, a second coupling part plate 255 and the second wire frame 256.

One side in the longitudinal direction of the second coupling part body 251 is coupled to the insertion tube 220, and the other side thereof is coupled to the first coupling part 150. At this instance, one side in the longitudinal direction of the second coupling part body 251 may be coupled to the other side in the longitudinal direction of the insertion tube 220, and the other side in the longitudinal direction of the second coupling part body 251 may be detachably coupled to the first coupling part body 151.

The second coupling part body 251 may have a diameter being reduced toward an end in the longitudinal direction (a tip end). As a result, one end in the longitudinal direction of the second coupling part body 251 may have a shape which can be coupled to the insertion tube part 224 of the insertion tube 220 and may be connected to the insertion tube 220.

The other side in the longitudinal direction of the second coupling part body 251 may be provided to be engaged with the first coupling part 150 to be coupled thereto. For example, the other side in the longitudinal direction of the second coupling part body 251 may have a plate shape of which three surfaces extend toward a direction of the manipulation part 100. As another example, the other side in the longitudinal direction of the second coupling part body 251 may have a shape of a pair of plates extending in a direction of the manipulation part 100. Accordingly, the second coupling part body 251 may be coupled to the first coupling part body 151 of the first coupling part 150.

As a result, the second coupling part body 251 and the first coupling part body 151 may be inserted into each other. That is, the second coupling part body 251 may be coupled to the first coupling part body 151 and may accommodate the first coupling plate 155, the first wire frame 156, the second coupling plate 255, and the second wire frame 256 on an inside thereof.

The second coupling plate 255 may be disposed between a pair of surfaces of the second coupling part body 251 facing each other. The second coupling plate 255 may be disposed on an inside of the second coupling part body 251 and may be disposed at a position facing the second coupling part body 251.

In addition, the second coupling plate 255 may be disposed at a position facing the first coupling plate 155. At this instance, when the first coupling plate 155 is moved along the movement of the rack gear 154, the first coupling plate 155 may be coupled to the second coupling plate 255.

For example, the second coupling plate 255 may take the form of a quadrangular plate having a certain thickness. At this instance, the fixing portion 255a for fixing coupling with the first coupling plate 155 may be formed in the second coupling plate 255. For example, the fixing portion 255a may have a shape of a protrusion, and may be coupled to the fixing portion 155a in a hole shape formed in the first coupling plate 155.

Meanwhile, the second wire frame 256 may be disposed on the second coupling plate 255. The second wire frame 256 may be coupled to the second coupling plate 255 to be able to rectilinearly reciprocate. For example, a guide groove configured to guide a rectilinear movement of the second wire frame 256 may be formed on the second coupling plate 255, and the second wire frame 256 may be disposed in the guide groove.

Meanwhile, the bending wire 230 may be coupled to the second wire frame 256. For example, the bending wire 230 may be coupled to one side in the longitudinal direction of the second wire frame 256, and the second wire frame 256 may be provided to be able to rectilinearly reciprocate along the longitudinal direction.

Meanwhile, the first wire frame 156 may be coupled to the second wire frame 256. For example, a frame coupling part 256b may be formed on the other side in the longitudinal direction of the second wire frame 256. The frame coupling part 256b may have a shape in which a plurality of ribs and grooves are formed alternately. The frame coupling part 256b may have a shape corresponding to a shape of the frame coupling part 156b. That is, the first wire frame 156 and the second wire frame 256 may be coupled to each other in a state of being engaged with each other.

An assembled body formed of the first wire frame 156 and the second wire frame 256 coupled to each other may be disposed inside a portion formed of the first coupling plate 155 and the second coupling plate 255 coupled to each other. At this instance, the first wire frame 156 may be accommodated in the guide groove formed in the first coupling plate 155, and the second wire frame 256 may be accommodated in the guide groove formed in the second coupling plate 255. Therefore, when the first coupling plate 155 and the second coupling plate 255 are coupled to each other, the first coupling plate 155 and the second coupling plate 255 coupled to each other may surround the assembled body formed of the first wire frame 156 and the second wire frame 256 coupled to each other. That is, the assembled body formed of the first wire frame 156 and the second wire frame 256 coupled to each other is capable of reciprocating along the longitudinal direction (forward and rearward directions), however, may be restrained on four surfaces in upward and rearward directions, and left and right directions by the first coupling plate 155 and the second coupling plate 255.

With this configuration, the manipulation wire 130 coupled to the first wire frame 156 and the bending wire 230 coupled to the second wire frame 256 may be moved in interrelation with each other, and may bend the insertion tube 220 according to the manipulation of the manipulation knob 120.

While the present invention has been described with reference to the specific embodiments, the specific embodiments are only for specifically explaining the present invention, and the present invention is not limited to the specific embodiments. It is apparent that the present invention may be modified or altered by those skilled in the art without departing from the technical spirit of the present invention.

All the simple modifications or alterations to the present invention fall within the scope of the present invention, and the specific protection scope of the present invention will be defined by the appended claims.

## Claims

1. An endoscope comprising:
an insertion part comprising a lighting imaging unit and an insertion tube; and
a manipulation part comprising a manipulation knob and a manipulation wire 130 configured to bend and operate the insertion tube according to manipulation of the manipulation knob,
**characterized in that** the manipulation part comprises:
a coupling part coupled to the insertion part according to a rotation of a dial provided to be rotatably operable.

2. The endoscope of claim 1,
wherein the coupling part further comprises:
a first coupling plate moved by a rotation of the dial to be coupled to the insertion part.

3. The endoscope of claim 2,
wherein the coupling part further comprises:
a pinion gear configured to be rotated in interrelation with the rotation of the dial; and
a rack gear coupled to the first coupling plate and configured to be engaged with the pinion gear and to reciprocate according to a rotation of the pinion gear

4. The endoscope of claim 2,
wherein the coupling part further comprises:
a first wire frame to which the manipulation wire is coupled and configured to reciprocate along the first coupling plate.

5. The endoscope of claim 4,
wherein the coupling part further comprises:
a locking pin disposed on the first coupling plate and accommodated in a fixing groove formed on the first wire frame according to a reciprocation.

6. The endoscope of claim 5,
wherein the coupling part further comprises:
a spring configured to pressurize the locking pin in a direction of the first wire frame.

7. The endoscope of claim 5,
wherein the coupling part further comprises:
a locking holder coupled to the first coupling plate and accommodating the locking pin to be capable of reciprocating.

8. The endoscope of claim 2,
wherein the insertion part comprises:
a second coupling plate detachably coupled to the first coupling plate.

9. The endoscope of claim 2,
wherein the insertion part comprises:
a second wire frame to which a bending wire is coupled,
the second wire frame being coupled to the first wire frame and configured to reciprocate together with the first wire frame.

10. An endoscope comprising:
an insertion part comprising a lighting imaging unit and an insertion tube; and
a manipulation part comprising a manipulation knob and configured to bend and operate the insertion tube according to manipulation of the manipulation knob,
**characterized in that** the manipulation part comprises:
a dial provided to be rotatably operable;
a pinion gear configured to be rotated in interrelation with the rotation of the dial;
a rack gear configured to be engaged with the pinion gear 13 and to reciprocate according to a rotation of the pinion gear; and
a coupling plate to which the rack gear is coupled and detachably coupled to the insertion part.

11. The endoscope of claim 10,
**characterized in that** the insertion part comprises:
a bending wire configured to bend and operate the insertion tube,
wherein the manipulation part comprises:
a manipulation wire configured to move the bending wire according to manipulation of the manipulation knob,
wherein the bending wire and the manipulation wire are detachably connected to each other according to an operation of the dial.
